Europäisches Patentamt

**(19)** European Patent Office

Office européen des brevets

**(11)** Veröffentlichungsnummer: **0 230 226 B1**

**(12)** EUROPÄISCHE PATENTSCHRIFT

**(45)** Veröffentlichungstag der Patentschrift: **07.08.91**

**(21)** Anmeldenummer: **87100102.0**

**(22)** Anmeldetag: **07.01.87**

**(51)** Int. Cl.5: **A61K 9/10**, A61K 47/00, A61K 31/415

Die Akte enthält technische Angaben, die nach dem Eingang der Anmeldung eingereicht wurden und die nicht in dieser Patentschrift enthalten sind.

**(54)** Antimykotische Emulsionen.

**(30)** Priorität: **15.01.86 DE 3600947**

**(43)** Veröffentlichungstag der Anmeldung:
**29.07.87 Patentblatt 87/31**

**(45)** Bekanntmachung des Hinweises auf die
Patenterteilung:
**07.08.91 Patentblatt 91/32**

**(84)** Benannte Vertragsstaaten:
**AT BE CH DE FR LI LU NL**

**(56)** Entgegenhaltungen:
**EP-A- 0 054 205**
**EP-A- 0 055 635**
**EP-A- 0 137 278**

**(73)** Patentinhaber: **Kali-Chemie Pharma GmbH
Hans-Böckler-Allee 20 Postfach 220
W-3000 Hannover 1(DE)**

**(72)** Erfinder: **Wischniewski, Martin, Dr.
Kattowitzer Weg 24
W-3057 Neustadt(DE)**
Erfinder: **Feicho, Lutz
Kantstrasse 20
W-3167 Burgdorf(DE)**

**(74)** Vertreter: **Lauer, Dieter, Dr.
c/o Kali-Chemie AG Postfach 220 Hans-
Böckler-Allee 20
W-3000 Hannover 1(DE)**

## Beschreibung

Die Erfindung betrifft neuartige antimykotische Emulsionen, die an sich bekannte Azolderivate, ein Lösungsmittel für diese Azolderivate sowie Wasser und übliche Formulierungshilfsmittel enthalten.

Für die Behandlung von Mykosen sind bereits Präparate in Emulsionsform bekannt und im Handel. Diese Präparate enthalten z.B. entsprechend DE-OS 30 45 913 als zwingenden Bestandteil Benzylalkohol als Lösungsmittel für den Wirkstoff. Der Einsatz von Benzylalkohol für medizinische Zwecke wird aber zunehmend kritisch beurteilt. Als weiterer zwingenden Bestandteil für eine gute Wirksamkeit der Emulsionen sehen beispielsweise die DE-OS 30 45 913 und DE-OS 31 06 635 den Zusatz von Spreitmitteln vor. Solche Rezepturen sind einerseits recht komplex zusammengesetzt, was einen beträchtlichen Aufwand in der Rohstoff-Lagerhaltung und bei Ansatz und Herstellung der Emulsionen bedingt. Zum anderen ist nicht sichergestellt, daß der Wirkstoff in diesen komplexen Systemen gelöst vorliegt, da er - wie beispielsweise aus DE-OS 32 43 544 oder DE-OS 33 21 043 hervorgeht - in solchen Emulsionen in fester Form vorliegt. Damit ist aber die Gefahr gegeben, daß der Wirkstoff bei längerer Lagerung sedimentiert oder auskristallisiert und zumindest Teile der Emulsion eine nur geringe Wirksamkeit aufweisen.

Die EP-A-O 137 228 beschreibt die Verwendung von Dimethylisosorbid und Diathylenglycol und dessen Ether als Lösungsmittel in Mitteln zur transdermalen Applikation von Arzneimittelwirkstoffen in denen der ArzneimittelwirkStoff zu mindestens 50% in einem nicht fließfähigen; physiologisch unbedenklichen Gel gelöst ist. welches in einem vernetzten Silikonelastomeren mikrodispers verteilt ist. Ziel dieser Zubereitung ist die Verbesserung der transdermalen Resorbierbarkeit von festen Arzneimitteln mit geringer Wasserlöslichkeit. Hinweise auf die Eignung der genannten Lösungsmittel für nur topisch zu verwendende Arzneimittelwirkstoffe wie antimykotisch wirksame Azol derivate werden in der EP-A-O 137 278 jedoch nicht gegeben.

Aufgabe der Erfindung ist es daher, eine Emulsion mit abgeändertem Lösungsmittel zur Verfügung zu stellen, bei der keine Wirkstoffsedimentation oder Kristallisation eintritt.

Diese Aufgabe wird durch eine Emulsion der in den Patentansprüchen beschriebenen Art gelöst.

Gegenstand der Erfindung sind also antimykotische Emulsionen enthaltend Azolderivate, Lösungsmittel, Wasser und übliche Formulierungshilfsstoffe, die dadurch gekennzeichnet sind, daß sie als Lösungsmittel einen Äther aus der Gruppe Diäthylenglycolalkyläther und 2,5-Dimethylisosorbid in einer Menge von 15 bis 70 Gew.-%, enthalten.

Die Verwendung dieser Äther als Lösungsmittel für antimykotisch wirksame Azolderivate ist neu.

Als Diäthylenglycolalkyläther kommen sowohl Dialkyl- als auch Monoalkyläther in Frage. Alkyl bedeutet dabei insbesondere Methyl, Äthyl, n-Propyl oder n-Butyl, wobei im Fall der Dialkyläther sowohl gleichartig als auch ungleichartig substituierte Äther in Frage kommen.

Bevorzugt ist die Verwendung von Diäthylenglycolmonoalkyläthern, wobei ganz besonders der Diäthylenglycolmonoäthyläther geeignet ist.

Je nach Art und Menge der zugesetzten üblichen Formulierungshilfsmittel kann der Gehalt an Lösungsmittel einerseits und der Gehalt an Wasser andererseits in weiten Bereichen variiert werden. Der Bereich für das Lösungsmittel kann von 15 bis 70 Gew.-% reichen, wobei auf die Gesamtmasse der Emulsion als 100 Gew.-% bezogen ist. Der Bereich für Wasser kann von 70 bis 10 Gew.-% vorzugsweise 60 bis 10 Gew.-%, reichen.

2,5-Dimethylisosorbid wird in einer bevorzugten Variante zu 15 bis 50 Gew.-% eingesetzt, während Wasser dann bevorzugt zu 70 bis 30 Gew.-% eingesetzt wird.

Bei Einsatz von Diäthylenglycolmonoalkyläther wird dieser bevorzugt zu 40 bis 60 Gew.-% eingesetzt. In diesem Fall beträgt die Wassermenge bevorzugt 50 bis 20 Gew.-%.

Bei der praktischen Ausgestaltung der Erfindung ist es vorteilhaft, Lösungsmittel und Wasser im Rahmen der angegebenen Absolutmengen untereinander in einem bestimmten Gewichtsverhältnis einzusetzen. Bewährt haben sich Verhältnisse von Diäthylenglycolalkyläther : Wasser im Bereich von 3 : 1 bis 1 : 2 vorzugsweise 2 : 1 bis 1 : 1,5, bzw. von 2,5-Dimethylisosorbid im Bereich von 6 : 1 bis 2 : 1 vorzugsweise 1,5 : 1 bis 1 : 1.

Die antimykotisch wirksamen Azolderivate sind an sich bekannt und werden beispielsweise in DE-PS 16 70 976, DE-PS 16 17 481, DE-OS 24 30 039 genannt. Bevorzugte Azolverbindungen sind jene, die unter dem Freinamen Clotrimazol, Trifonazol, Lombazol bekannt sind. Gute Resultate werden mit Wirkstoffkonzentrationen von 0,5 bis 5 Gew.-% erzielt.

Zur Bereitung der erfindungsgemäßen Emulsionen können übliche pharmazeutische Formulierungshilfsstoffe aus einer oder mehreren der folgenden Gruppen zugesetzt werden.

Emulgatoren, insbesondere nichtionogene Emulgatoren wie beispielswise Sorbitanfettsäureester, Polyoxyäthylen-Fettsäureester, Polyoxyäthylen-Fettalkoholäther;

Salbengrundlagen wie beispielsweise Mono- und/oder Di-Glyceride mittelkettiger ($C_6$-$C_{12}$) oder langkettiger ($C_{14}$ bis $C_{22}$) Fettsäuren, Lanolin, Wollwachsalkohol-Salbe.

Ferner können ebenfalls übliche Konservierungsmittel, wie z.B. Phenoxyäthanol, Extender, farbgebende Substanzen und/oder Parfümierungsmittel zugesetzt werden.

Überraschenderweise kann bei Verwendung der erfindungsgemäß vorgesehenen Lösungsmittel das ansonsten notwendige Spreitmittel ohne Nachteile fortgelassen werden. Aus dem Stand der Technik war nicht zu erwarten, daß der Austausch eines unerwünschten Lösungsmittels gleichzeitig zu einer Rezepturvereinfachung führt, bei dem ein bisher als wesentlich angesehener Bestandteil fortfallen kann und ausserdem Emulsionen entstehen, die keinerlei nachteilige Sedimentation oder Kristallisation aufweisen.

Die Emulsionen können zur Anwendung in üblicher Weise appliziert werden. Sie können beispielsweise als Creme direkt auf die zu behandelnde Stelle aufgebracht und verteilt werden. Es ist aber auch möglich, flüssige Formen der Emulsion bei entsprechender Zusammensetzung direkt oder als Schaumaerosol zu applizieren.

Für die Herstellung der Emulsionen ist es vorteilhaft, Wasser und die Formulierungshilfsmittel·bei 50 bis 80°C zu vereinigen und zu diesem Stammansatz die Lösung des Azolderivats im Lösungsmittel bei der angegebenen Temperatur oder vorzugsweise bei 30 bis 50°C zuzusetzen.

Die folgenden Beispiele sollen die Erfindung näher erläutern.

Beispiel

| Bestandteil | | a | b | c | d |
|---|---|---|---|---|---|
| I. | Nichtiogene Emulgatoren[1] | 3,5 | 3,5 | 3,5 | 3,5 |
| | Salbengrundlage[2] | 13,0 | 13,0 | 13,0 | 13,0 |
| | Konservans | – | 0,18 | – | – |
| II. | entmineralisiertes Wasser | 31,25 | 31,16 | 22,5 | 68,0 |
| III. | Diäthylenglycolmonoäthyläther | 51,25 | 51,16 | 60,0 | – |
| | 2,5 Dimethylisosorbid | – | – | – | 14,5 |
| | Clotrimazol | 1,0 | 1,0 | 1,0 | 1,0 |

[1]  2,0 g Span 60 + 1,5 g Tween 60
[2]  3,0 g Cetylpalmitat + 10,0 g Lanette N

Phase I wird bei 75°C geschmolzen, Phase 11 auf 75°C erhitzt und in Phase I unter Rühren eingearbeitet. Es wird bis 40°C kaltgerührt und dann Phase III eingearbeitet. Die Emulsion wird anschließend bis 25°C kaltgerührt. Man erhält eine glatte Creme.

## Patentansprüche

1. Antimykotische Emulsionen enthaltend Azolderivate, Lösungsmittel, Wasser und übliche Formulierungshilfsstoffe, dadurch gekennzeichnet, daß sie als Lösungsmittel einen Äther aus der Gruppe Diäthylenglycolalkyläther und 2,5-Dimethylisosorbid in einer Menge von 15 bis 70 Gew.-%, enthalten.

2. Emulsionen gemäß Anspruch 1, dadurch gekennzeichnet, daß sie als Lösungsmittel Diäthylenglycolmonoalkyläther in einer Menge von 40 bis 60 Gew.-% enthalten.

EP 0 230 226 B1

3. Emulsionen gemäß Anspruch 1 oder 2, dadurch gekennzeichnet, daß Alkyl die Bedeutung Methyl, Äthyl, n-Propyl, n-Butyl, vorzugsweise Äthyl, besitzt.

4. Emulsionen gemäß einem der Ansprüche 1 bis 3, dadurch gekennzeichnet, daß sie 60 bis 10 Gew.-%, vorzugsweise 50 bis 20 Gew.-% Wasser enthalten.

5. Emulsionen gemäß einem der Ansprüche 1 bis 4, dadurch gekennzeichnet, daß sie Diäthylenglycolalkyläther und Wasser im Gewichtsverhältnis 3:1 bis 1:2, vorzugsweise 2:1 bis 1:1,5 enthalten.

6. Emulsionen gemäß Anspruch 1, dadurch gekennzeichnet, daß sie als Lösungsmittel 15 bis 50 Gew.-% 2,5-Dimethylisosorbid enthalten.

7. Emulsionen gemäß einem der Ansprüche 1 bis 6, dadurch gekennzeichnet, daß sie als Azolderivate einen Wirkstoff aus der Gruppe Clotrimazol, Trifonazol, Lombazol enthalten.

8. Emulsionen gemäß einem der Ansprüche 1 bis 7, dadurch gekennzeichnet, daß sie ein oder mehrere Formulierungshilfsstoffe aus der Gruppe Emulgatoren, Salben-grundlage, Konservierungsmittel enthalten.

9. Verfahren zur Herstellung von antimykotischen Emulsionen enthaltend Azolderivate, Lösungsmittel, Wasser und übliche Formulierungshilfsstoffe, dadurch gekennzeichnet, daß man als Lösungsmittel einen Äther aus der Gruppe Diäthylenglycolalkyläther und 2,5-Dimethylisosorbid in einer Menge von 15 bis 70 Gew.-%, verwendet.

## Claims

1. Antimycotic emulsions containing azole derivatives a solvent, water and conventional auxiliary formulating agents, characterized in that they contain as the solvent ethers from the group of diethylene glycol alkyl ethers and 2,5-dimethylisosorbide in an amount of 15 to 70 % by weight.

2. Emulsions according to claim 1 characterized in that they contain as the solvent diethylene glycol monoalkylethers in an amount of 40 to 60 % by weight.

3. Emulsions according to claim 1 or 2 characterized in that alkyl denotes methyl, ethyl, n-propyl, n-butyl, preferably ethyl.

4. Emulsions according to one of the claims 1 to 3, characterized in that they contain 60 to 10 % by weight, preferably 50 to 20 % by weight of water.

5. Emulsions according to one of the claims 1 to 4, characterized in that they contain diethylene glycol alyylethers and water in a per weight ratio of 3 : 1 to 1 : 2, preferably 2 : 1 to 1 : 1,5.

6. Emulsions according to claim 1, characterized in that they contain as the solvent 15 to 20 % by weight of 2,5-dimethylisosorbide.

7. Emulsions according to one of the claims 1 to 6, characterized in that they contain as an azole derivative an active compound from the group of clotrimazole. trifonazole. lombazole.

8. Emulsions according to one of the claims 1 to 7. characterized in that they contain one or more auxiliary formulating agents from the group of emulsifiers, ointment base materials, preservatives.

9. Process for preparing antimycotic emulsions containing azole derivatives a solvent, water and conventional auxiliary formulating agents, characterized in that ethers from the group of diethylene glycol alkylethers and 2,5-dimethylisosorbide are used as the solvent in an amount of 15 to 70 % by weight.

## Revendications

1. Emulsions antimycosiques contenant des dérivés d'azoles, un solvant, de l'eau et des adjuvants de

4

formulation usuels, caractérisées en ce qu'elles contiennent en tant que solvant un éther choisi parmi un éther alkylique de diéthylèneglycol et le 2,5-diméthylisosorbide, en une quantité de 15 à 70 % en poids.

2. Emulsions selon la revendication 1, caractérisées en ce qu'elles contiennent en tant que solvant un éther monoalkylique de diéthylèneglycol en une quantité de 40 à 60 % en poids.

3. Emulsions selon la revendication 1 ou 2, caractérisées en ce que le groupe alkyle signifie le groupe méthyle, éthyle, n-propyle, n-butyle, de préférence le groupe éthyle.

4. Emulsions selon l'une des revendications 1 à 3, caractérisées en ce qu'elles contiennent de 60 à 10 % en poids, de préférence de 50 à 20 % en poids d'eau.

5. Emulsions selon l'une des revendications 1 à 4, caractérisées en ce qu'elles contiennent un éther alkylique de diéthylèneglycol et de l'eau en un rappport pondéral de 3:1 à 1:2, de préférence de' 2:1 à 1:1,5.

6. Emulsions selon la revendication 1, caractérisées en ce qu'elles contiennent en tant que solvant de 15 à 50 % en poids de 2,5-diméthylisosorbide.

7. Emulsions selon l'une des revendications 1 à 6, caractérisées en ce qu'elles contiennent en tant que dérivés d'azolesune substance active choisie parmi le clotrimazole, le trifonazole, le lombazole.

8. Emulsions selon l'une des revendications 1 à 7, caractérisées en ce qu'elles contiennent un ou plusieurs adjuvants de formulation choisis parmi des émulsifiants, des excipients pour pommade, des agents de conservation.

9. Procédé pour la préparation d'émulsions antimycosiques contenant des dérivés d'azoles, un solvant, de l'eau et des adjuvants de formulation usuels, caractérisé en ce que l'on utilise en tant que solvant un éther choisi parmi un éther alkylique de diéthylèneglycol et le 2,5-diméthylisosorbide, en une quantité de 15 à 70 % en poids.